# EUROPÄISCHE PATENTSCHRIFT

(11) **EP 0 706 358 B1**
(45) Veröffentlichungstag und Bekanntmachung des Hinweises auf die Patenterteilung: **25.02.1998**
(21) Anmeldenummer: 94922252.5
(22) Anmeldetag: 01.07.1994
(51) Int. Cl.: A61F 13/06

(54) **GELENKBANDAGE**
JOINT BAND
GENOUILLERE

(30) Priorität: 02.07.1993 DE 4322028
(43) Veröffentlichungstag der Anmeldung: 17.04.1996
(73) Patentinhaber: FERD. HAUBER GmbH & CO. KG, D-72622 Nürtingen (DE)
(72) Erfinder: GNEITING, Günter, D-72622 Nürtingen (DE)
(74) Vertreter: Becker, Maria, Dipl.-Phys.
(86) Internationale Anmeldenummer: EP9402163
(87) Internationale Veröffentlichungsnummer: WO9501145

(56) Entgegenhaltungen:
- WO-A-94/07443
- DE-A- 4 103 386
- DE-C- 4 129 675
- US-A- 1 727 897
- US-A- 4 492 227

## Beschreibung

Die Erfindung betrifft eine Gelenkbandage nach dem Oberbegriff des Anspruchs 1.

Bei einer derartigen bekannten Gelenkbandage (DE 41 03 386 A1), die von einem strumpfförmigen Schlauch aus elastischem, dehnbarem Material gebildet ist, ist im Gelenkbereich, in dem bei angelegter Bandage aufgrund der Gelenkbewegungen die grösste Dehnung der Bandage auftritt, ein Einsatz eingearbeitet, der aus einer Deckstruktur aus verhältnismässig unelastischem Garn und aus einer elastischen Fadenanordnung gebildet ist, die der Deckstruktur eine Querwellenstruktur verleiht, sie stabilisiert und vorspannt.

Dieser Einsatz ist hauptsächlich der Aussenseite des jeweiligen Gelenks zugeordnet und soll eine Faltenbildung der Bandage im Beugebereich vermeiden.

Nachteilig bei dieser bekannten Bandage ist es, daß bei bestimmungsgemäß angelegter Bandage sich im Beugebereich insbesondere bei abgewinkeltem Gelenk ein Stoffwulst bildet, der für den Benutzer der Bandage unangenehm und störend ist, und zwar selbst dann, wenn im Beugebereich der Bandage nur ein schmaler oder gar kein Einsatz vorhanden ist.

Aus der US-A-4492227 ist eine Kniebandage aus elastischem textilen Schlauchmaterial bekannt, die aus drei Abschnitten besteht, wobei der mittlere, im Kniebereich liegende Abschnitt eine 2 bis 10-fach höhere Dehnbarkeit aufweist als die äußeren Abschnitte. Diese höhere Dehnbarkeit wird bewirkt durch eine dichtere Strickart oder den Einsatz anderer Materialien. Weiter weist diese Bandage in einem Winkel zur Längsachse verlaufende Stabilisierungsbänder auf. Hierdurch wird zwar eine gewisse Stabilisierung im Kniebereich erreicht, nicht aber der Nachteil beseitigt, daß sich beim Beugen des Kniegelenks in der Kniekehle Falten bilden. Zur Reduzierung der Faltenbildung ist ein in Bandagenlängsrichtung verlaufender Schlitz im Kniekehlenbereich vorgesehen, der in einer Naht der Bandage vorgesehen sein kann.

DE-A 4129675 beschreibt eine Epicondylitisbandage aus elastischem Schlauchmaterial, bei der in der Ellenbeuge ein faltenbalgartig ausgebildetes Übergangsstück, z.B. aus Schaumstoff, vorgesehen ist, um eine Beugung des Gelenks zu erleichtern. Dieses faltenbalgartige Übergangsstück vermittelt jedoch ein unangenehmes Tragegefühl, insbesondere wenn nach längerem Tragen, bedingt durch die Körperfeuchte, sich harte Wülste bilden.

Demgegenüber liegt der Erfindung die Aufgabe zugrunde, eine Gelenkbandage der eingangs genannten Art zu schaffen, bei der die Bildung eines störenden Stoffwulstes beim Abwinkeln des Gelenks in der Kniekehle des Trägers vermieden wird, ohne daß die Fixierung des Gelenks im Beugebereich und das Tragegefühl beeinträchtigt wird.

Zur Lösung dieser Aufgabe dienen, ausgehend von einer Gelenkbandage gemäß dem Oberbegriff des Anspruchs 1, dessen kennzeichnende Anspruchsmerkmale.

Erfindungsgemäß wird also in dem der Gelenkbeuge zugeordneten Bereich der Bandage ein Einsatz über ¼ bis ½ des Schlauchumfangs vorgesehen, der nahtlos in den Schlauch eingearbeitet ist und der aus einem dünneren und elastischeren sowie eine andere Bindungsstruktur als der Schlauch aufweisenden Material besteht, dessen Dehnbarkeit etwa doppelt so groß ist wie die des textilen Schlauchmaterials. Durch diese erheblich größere Dehnbarkeit des Einsatzes im Gelenkbeugenbereich wird nicht nur eine Faltenbildung in der Gelenkkehle vermieden, sondern es wird auch das Strecken des Gelenks erleichtert. Der Vorteil des dünneren, elastischeren und weicheren Materials in der Gelenkbeuge wird nicht durch störende Nähte wieder zunichte gemacht.

Zweckmässige Weiterbildungen der Erfindung sind in den Ansprüchen 3 und 4 beschrieben.

Besonders vorteilhaft ist es, wenn der Einsatz im Schlauch einen genau festgelegten Rand aufweist und der Einsatz insbesondere etwa linsenförmig ist. Hierdurch lässt sich der für die Stabilisierung des Gelenks insbesondere im Bereich der Beugeachse erforderliche Bandagendruck trotz des Einsatzes aus dünnerem und elastischerem Material besonders gut aufrechterhalten, da die umfangsmässigen Kräfte, die von der Bandage zur Stabilisierung des Gelenks ausgeübt werden, im an den Einsatz angrenzenden Randbereich des normalen Bandagenmaterials geführt werden.

Die Erfindung wird im folgenden beispielsweise anhand der Zeichnung näher erläutert. In dieser zeigen:
- Fig. 1: eine schematische Seitenansicht einer Gelenkbandage, die z.B. an einem Knie angelegt ist,
- Fig. 2: eine Rückansicht der Bandage nach Fig. 1.

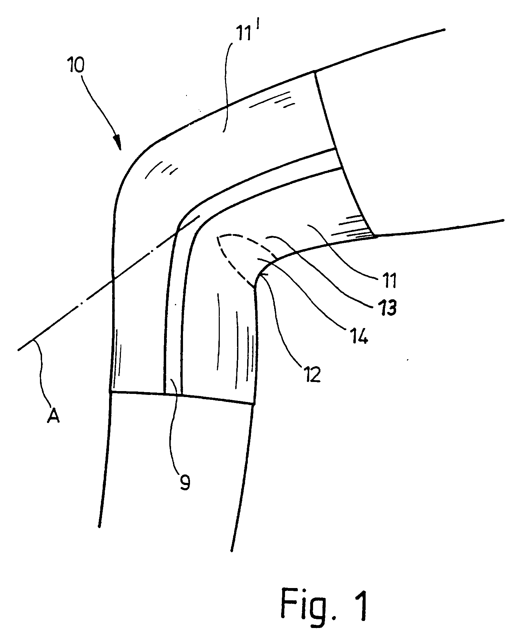

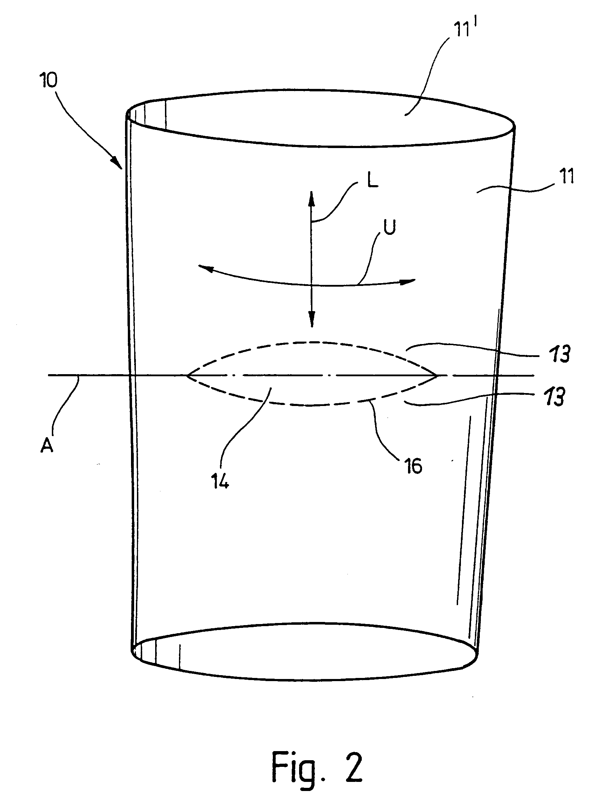

Die in Fig.1 und 2 gezeigte Gelenkbandage, die als Kniebandage ausgebildet ist, weist einen strumpfförmigen Schlauch 10 auf, der aus einer hinteren und einer vorderen Bandagenstofflage 11 bzw. 11' besteht. Die beiden Bandagenstofflagen 11, 11' sind dabei an ihren Seitenlängskanten miteinander verbunden.

Wird als Bandagenstoff vorzugsweise ein elastisches, dehnbares Gestrick verwendet, so erfolgt die Verbindung der beiden Bandagenstofflagen 11, 11' insbesondere durch Vernähen, wobei z.B. ein zusätzlicher Nahtstreifen 9 vorgesehen sein kann.

Die den hinteren Schlauchbereich bildende Bandagenstofflage 11 weist im Gelenkbereich, also im Bereich der Gelenkbeuge 12, einen Einsatz 14 auf, der aus einem dünneren und elastischeren Material besteht als die restlichen Bandagenstofflagen.

Der im Bereich der Beugeachse A des Kniegelenks liegende Einsatz 14 besitzt eine durch einen Rand 16 äusserlich scharf abgegrenzte Fläche, die etwa linsenförmig ausgebildet ist. Die Länge des Einsatzes 14 in Umfangsrichtung U des Schlauchs 10 beträgt dabei zwischen 1/4 und 1/2, vorzugsweise 1/3, des Schlauchumfangs im Gelenkbereich. Die Länge ist also so an die Dicke des Gelenks in Richtung der Beugeachse A angepasst, dass sich der Einsatz bis in den Seitenbereich des Gelenks erstreckt.

Zur Herstellung der hinteren Bandagenstofflage 11, deren textiles Material eine Maschenware ist, wird der Einsatz als Intarsia-Strickfläche eingearbeitet. Der Einsatz wird dabei mit einer anderen Stricktechnik und aus einem anderen Material gebildet, so dass der Einsatz nahtlos, aber scharf abgegrenzt in die hintere Bandagenstofflage 11 eingestrickt ist.

Beim Tragen der sachgemäss angelegten Gelenkbandage wird eine Faltenbildung durch den in der Gelenkbeuge angeordneten Einsatz 14 verhindert, da sich dieser beim Strecken des Gelenks besser ausdehnt, als dass es das übrige Bandagenmaterial tun würde, so dass hierdurch ein Hineinziehen der Bandage in die Gelenkbeuge verhindert wird. Beim Abbiegen zieht sich das Material des Einsatzes wieder zusammen, so dass keine Faltenbildung entsteht. Aufgrund des dünneren und weichen Materials und die Vermeidung einer Faltenbildung wird der Tragekomfort der erfindungsgemässen Gelenkbandage wesentlich erhöht.

Die erfindungsgemässe Gelenkbandage, die sich auf wirtschaftliche Weise herstellen lässt und insbesondere als Knie- oder Ellenbogenbandage Verwendung findet, kann in an sich üblicher Weise mit geeigneten Druckpolstern od.dgl. ausgestattet werden, wie es der jeweilige Anwendungszweck erfordert.

## Patentansprüche

1. Gelenkbandage mit einem strumpfförmigen Schlauch (10) aus elastischem, dehnbaren Textilmaterial, wobei der Schlauch (10) im Gelenkbereich (12) einen Einsatz (14) aufweist, der eine größere Dehnbarkeit aufweist, als das übrige Schlauchmaterial,und wobei der Einsatz (14) aus einem dünneren, elastischeren Material besteht, dessen Dehnbarkeit etwa doppelt so groß ist, wie die des textilen Schlauchmaterials,
**dadurch gekennzeichnet,**
daß sich der Einsatz (14) über ¼ bis ½ des Schlauchumfangs erstreckt und nahtlos in den Schlauch (10) eingearbeitet ist.

2. Gelenkbandage, nach Anspruch 1, dadurch gekennzeichnet, daß sich der Einsatz (14) über 1/3 des Schlauchumfangs erstreckt.

3. Gelenkbandage, nach Anspruch 1 oder 2, dadurch gekennzeichnet, daß der Einsatz (14) im Schlauch (10) einen genau festgelegten Rand (16) aufweist.

4. Gelenkbandage, nach einem der vorhergehenden Ansprüche, dadurch gekennzeichnet, daß der Einsatz (14) etwa linsenförmig ist.

## Claims

1. Joint band comprising a stocking-shaped hose (10) made from an elastic, extensible textile material, with an insert (14) arranged in the joint area (12) of the hose (10), that exhibits a greater extensibility than the remaining hose material, the insert (14) consisting of a thinner, more elastic material that is at least twice as extensible as the textile hose material,
**characterized in that**
the insert (14) extends over ¼ to ½ of the circumference of the hose and is seamlessly integrated into the hose (10).

2. Joint band according to claim 1, characterized in that the insert (14) extends over 1/3 of the circumference of the hose.

3. Joint band according to claim 1 or 2, characterized in that the insert (14) in the hose (10) has a precisely fixed edge (16) in the hose (10).

4. Joint band according to any of the preceding claims, characterized in that the insert (14) has a substantially lenticular shape.

## Revendications

1. Bandage pour articulation avec une gaine en forme de bas (10) en matériau textile élastique extensible, la gaine (10) présentant dans la zone de l'articulation (12) un gousset (14) présentant une extensibilité plus élevée que le reste du matériau de la gaine, et le gousset (14) consistant en un matériau élastique plus mince, dont l'extensibilité est environ deux fois plus élevée que celle du matériau textile de la gaine,
**caractérisé en ce que**
le gousset (14) s'étend sur ¼ à ½ de la circonférence de la gaine et est intégré sans couture dans la gaine (10).

2. Bandage pour articulation selon la revendication 1, caractérisé en ce que le gousset (14) s'étend sur 1/3 de la circonférence de la gaine.

3. Bandage pour articulation selon la revendication 1 ou 2, caractérisé en ce que le gousset (14) présente un bord (16) exactement défini dans la gaine (10).

4. Bandage pour articulation, selon l'une des revendications précédentes, caractérisé en ce que le gousset (14) est à peu près lenticulaire.
